# EUROPÄISCHE PATENTSCHRIFT

(11) **EP 0 481 395 B1**
(45) Veröffentlichungstag und Bekanntmachung des Hinweises auf die Patenterteilung: **25.01.1995**
(21) Anmeldenummer: 91117470.4
(22) Anmeldetag: 14.10.1991
(51) Int. Cl.: C07C 67/343, C07C 69/716, C07C 69/738

(54) **Verfahren zur Herstellung von 3-Oxocarbonsäureestern**
Process for the preparation of esters of 3-oxocarboxylic acids
Procédé de préparation d'esters d'acides 3-cétocarboxyliques

(30) Priorität: 15.10.1990 CH 3301/90
(43) Veröffentlichungstag der Anmeldung: 22.04.1992
(73) Patentinhaber: LONZA AG, CH-3945 Gampel/Wallis (CH)
(72) Erfinder: Eyer, Martin, Dr., GLIS (Kanton Wallis) (CH)
(74) Vertreter: Weinhold, Peter, Dr.

(56) Entgegenhaltungen:
- C. R. HAUSER et al,"ORGANIC REACTIONS", 1942, John Wiley & Sons, New York, US, Band 1, S. 297-302
- H. HENECKA,"HOUBEN WEYL, METHODEN DER ORGANISCHEN CHEMIE", 1952, Georg Thieme Verlag, Stuttgart, DE, Band VIII, S. 615-616

## Beschreibung

Gegenstand der Erfindung ist ein Verfahren zur Herstellung von 3-Oxocarbonsäureestern aus Acetessigsäureestern.

3-Oxocarbonsäureester (Acylessigsäureester) der allgemeinen Formel 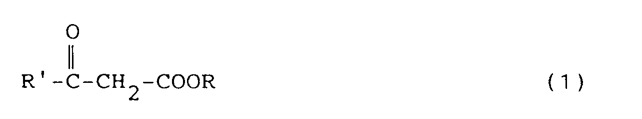
sind ausserordentlich wichtige und vielseitig verwendbare Zwischenprodukte. Dies ist auf die zahlreichen verschiedenen Reaktionsmöglichkeiten dieser Verbindungen zurückzuführen.
So ergibt ihre Kupplung mit aromatischen Diazoniumsalzen 2-(Arylhydrazono)-3-oxocarbonsäureester, die als Farbstoffe beispielsweise in photographischen Materialien zum Einsatz gelangen.
Mit Hydrazinen cyclisieren sie zu Pyrazolonen, von denen einige wertvolle pharmazeutische Wirkstoffe sind. Halogenierte Benzoylessigsäureester (R' = substit. Phenyl) sind Ausgangsprodukte für Chinolon- und Cinnolincarbonsäuren, die beispielsweise als Antibiotika (z.B. Ciprofloxacin) eingesetzt werden können.
3-Oxopentansäureester (R' = Ethyl) ist ein wichtiges Zwischenprodukt für die Synthese des entzündungshemmenden Wirkstoffs Etodolac.
Es sind schon seit längerer Zeit zahlreiche Methoden zur Herstellung von 3-Oxocarbonsäureestern bekannt. Eine Uebersicht geben z.B. C.R. Hauser und B.E. Hudson Jr. in Organic Reactions Vol.1 (1942), S.297-302.
Allgemeiner anwendbar ist insbesondere die Acylierung von Malonestern oder Acetessigsäureestern mit Carbonsäurechloriden mit nachfolgender Abspaltung einer Estergruppe bzw. einer Acetylgruppe (siehe z.B. G.Hesse in "Methoden der organischen Chemie", 4.Aufl., Bd.VI/1d (1978), S.73).
Aus Kostengründen ist für die Produktion im technischen Massstab Acetessigsäureester als Ausgangsmaterial vorzuziehen. Vorteilhaft wird dabei der Acetessigsäureester in Form eines Metallenolats eingesetzt. Hierfür eignen sich besonders die Magnesiumenolate, die sogar in wässrigem Medium leicht herstellbar sind (siehe z.B. M.Conrad, Justus Liebigs Ann.Chem. 181, 272 (1877)).
Ein weiterer Vorteil der Verwendung von Acetessigsäureestern anstelle von Malonestern als Ausgangsmaterial ist die gegenüber den Acylmalonestern einfachere Spaltung der als Zwischenprodukt entstehenden 2-Acyl-acetessigsäureester (siehe z.B. H.Henecka in "Methoden der organischen Chemie", 4.Aufl., Bd.VIII (1952), S.615f), die beispielsweise mit wässrigem Ammoniak erfolgen kann.

Es hat sich jedoch gezeigt, dass die Acylierung von Acetessigsäureester-magnesiumenolat mit Carbonsäurechloriden in der Praxis oft zu unbefriedigenden Resultaten führt. So werden insbesondere oft Nebenprodukte wie beispielsweise Diacetessigester erhalten, die nicht nur die Ausbeute an gewünschten Produkt herabsetzen, sondern auch noch schwierig abtrennbar sind und dadurch zu unvertretbarem Aufwand bei der Aufarbeitung führen oder die Gewinnung eines reinen Produktes gänzlich unmöglich machen.
Beispielsweise ergab die Nacharbeitung des in der veröffentlichten japanischen Patentanmeldung JP 57-70837 beschriebenen Verfahrens zur Herstellung von Pivaloylessigsäureethylester aus Acetessigester-magnesiumenolat und Pivaloyl-Chlorid nur nutzbare Ausbeuten von ca. 35% und ein sehr unreines Produkt mit einem Gehalt von nur ca. 70%.

Aufgabe der vorliegenden Erfindung war daher, ein Verfahren zur Herstellung von 3-Oxocarbonsäureestern der allgemeinen Formel 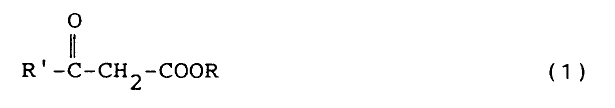
aus den entsprechenden Acetessigsäureester-magnesiumenolaten und den entsprechenden Carbonsäurechloriden zur Verfügung zu stellen, welches für eine grosse Anzahl verschiedenartiger Reste R und R' die gewünschten Produkte in hoher Ausbeute und Reinheit liefert und in technischem Massstab kostengünstig durchführbar ist.

Erfindungsgemäss wird diese Aufgabe durch das Verfahren nach Patentanspruch 1 gelöst.

Es wurde gefunden, dass durch Zugabe eines tertiären Amins bei der Umsetzung des Acetessigsäureester-magnesiumenolats mit dem Carbonsäurechlorid die Ausbeute erheblich gesteigert und die Bildung unerwünschter Nebenprodukte weitgehend unterdrückt werden kann.
Als tertiäres Amin sollen hier im wesentlichen alle Verbindungen gelten, die wenigstens ein basisches Stickstoffatom besitzen und am Stickstoff keine Wasserstoffatome tragen. Hierzu zählen insbesondere:
Trialkylamine mit gleichen oder verschiedenen geradkettigen, verzweigten oder cyclischen Alkylresten, wie beispielsweise Trimethylamin, Triethylamin, Tripropylamin, Tributylamin, Triisobutylamin, Diethyl-methylamin, Dimethyl-cyclohexyl-amin, Alkyl-(arylalkyl)amine, wie beispielsweise N,N-Dimethylbenzylamin, Aryl-alkyl-amine, wie beispielsweise N,N-Dimethylanilin oder N,N-Diethylanilin, Verbindungen, in denen der Stickstoff in ein gesättigtes Ringsystem eingebaut ist, wie beispielsweise N-Alkylpyrrolidine, N-Alkylpiperidine, N-Alkylmorpholine, Chinuclidin, 1,4-Diazabicylo[2.2.2]octan ("DABCO"), oder auch aromatische Stickstoffheterocyclen wie beispielsweise Pyridin, Chinolin, Alkylpyridine wie Methyl-, Methylethyl-, Dimethyl- und Trimethylpyridine, 4-(Dialkylamino)pyridine wie 4-(Dimethylamino)pyridin ("DMAP"), sowie amidinartige Verbindungen wie 1,5-Diazabicyclo[4.3.0]non-5-en ("DBN") und 1,8-Diazabicyclo[5.4.0]undec-7-en ("DBU"). Vorzugsweise wird ein Trialkylamin eingesetzt, besonders bevorzugt ist Triethylamin.
Das tertiäre Amin wird zweckmässig in stöchiometrischen Mengen, vorzugsweise in Mengen von 1,0 bis 1,4 mol, bezogen auf 1 mol des Magnesiumenolats, zugegeben.
Als Ausgangsmaterial für das erfindungsgemässe Verfahren eignen sich im wesentlichen alle Acetessigsäureester der allgemeinen Formel 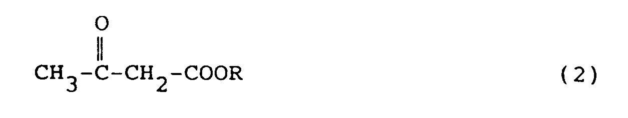
die hinreichend stabile Magnesiumenolate bilden. Dies sind insbesondere die Ester geradkettiger oder verzweigter primärer, sekundärer oder tertiärer Alkanole mit bis zu 20 C-Atomen, worin R in der allgemeinen Formel 2 entsprechend ein geradkettiger oder verzweigter Alkylrest mit 1-20 C-Atomen ist, beispielsweise Methyl, Ethyl, Butyl, Hexyl, Dodecyl, Octadecyl, Isopropyl, Isobutyl oder tert-Butyl, die Ester ungesättigter Alkohole mit 3 bis 6 C-Atomen, worin R beispielsweise Allyl, β-Methallyl, Crotyl, 4-Penten-1-yl, oder Propargyl ist, die Ester cyclischer Alkohole, worin R beispielsweise Cyclohexyl ist, die Ester von phenylsubstituierten C₁-C₄-Alkanolen und -Alkenolen, worin R beispielsweise Benzyl, Phenylethyl oder Cinnamyl ist, die Ester von Ethylenglykolmonoalkylethern mit C₁-C₄-Alkylgruppen, worin R beispielsweise 2-Methoxyethyl ist, sowie die Ester von gegebenenfalls substituierten Phenolen, worin R beispielsweise Phenyl, Tolyl oder Anisyl ist. Als Säurechloride sind im wesentlichen alle Carbonsäurechloride der allgemeinen Formel R'-COCl (3) mit Ausnahme von unsubstituierten Acetylchlorid verwendbar, insbesondere geradkettige oder verzweigte Alkanoylchloride mit bis zu 21 C-Atomen, worin R' beispielsweise Ethyl, Propyl, Butyl, Hexyl, Octyl, Dodecyl, Octadecyl, Isopropyl, Isobutyl, tert-Butyl oder Neopentyl ist, Alkanoylchloride, die durch Halogene, niedrige Alkoxygruppen, niedrige Alkylthiogruppen, gegebenenfalls substituierte Arylgruppen, Cycloalkylgruppen, Aryloxygruppen, Arylthiogruppen oder gesättigte oder ungesättigte heterocyclische Reste substituiert sind, worin also R' beispielsweise 2-Chlorethyl, Methoxymethyl, Methylthiomethyl, Benzyl, Phenethyl, p-Methoxybenzyl, p-Methylbenzyl, Cyclohexylmethyl, Phenoxymethyl, Phenylthiomethyl, Pyridylmethyl, Tetrahydrofurylmethyl ist, geradkettige oder verzweigte Alkenoylchloride mit bis zu 21 C-Atomen, worin R' beispielsweise Vinyl, Allyl, Isopropenyl, Methallyl, Crotyl oder 8-Heptadecen-1-yl ist, Alkenoylchloride, die durch Halogene oder Arylgruppen substituiert sind, worin R' also beispielsweise 1-Chlorvinyl, 2-Phenylethenyl, 2-(4-Chlorphenyl)ethenyl ist, gegebenenfalls durch Arylreste substituierte Alkinoylchloride, worin R' beispielsweise Ethinyl, 1-Propinyl, 2-Propinyl oder Phenylethinyl ist, gegebenenfalls substituierte Cycloalkanoyl- oder Cycloalkenoylchloride, worin R' beispielsweise Cyclopropyl, Cyclobutyl, Cyclopentyl, Cyclohexyl, 2,2-Dimethylcyclopropyl, Menthyl, 1-Cyclohexenyl ist, gegebenenfalls substituierte mono- oder polycyclische Aroylchloride, worin R' beispielsweise Phenyl, o-, m- oder p-Tolyl, Xylyl, Mesityl, p-Methoxyphenyl, p-Chlorphenyl, 2,4,6-Trifluorphenyl, 1-Naphthyl oder 2-Naphthyl ist, Chloride gesättigter oder ungesättigter heterocyclischer Carbonsäuren, worin R' beispielsweise 2-Pyridyl, 3-Pyridyl, 4-Pyridyl, 6-Chlor-3-pyridyl, 6-Hydroxy-3-pyridyl, 5,6-Dichlor-3-pyridyl, 6-Methyl-3-pyridyl, 4-Piperidyl, 2-Furyl, 2-Thienyl, 2-Pyrrolidinyl, 2-Indolyl ist.

Sofern hier keine anderslautende Präzisierung des Begriffs Alkylgruppen (als solche oder als Bestandteil größerer Gruppen) gegeben ist, enthalten diese vorzugsweise 1 bis 6, insbesondere 1 bis 4 Kohlenstoffatome. Dieselbe Bedeutung hat auch der Ausdruck "niedrige" im Zusammenhang mit Alkoxy- bzw. Alkylthiogruppen. Die Arylgruppe ist vorzugsweise eine Phenylgruppe. Unter Halogensubstituenten werden z.B. Chlor-, Brom- oder Fluorgruppen verstanden. Beispiele von Substituenten von gegebenenfalls substituierten Gruppen sind niedrige Alkylgruppen, Halogengruppen oder niedrige Alkoxygruppen.

Das Säurechlorid wird zweckmäßig in der stöchiometrisch erforderlichen Menge oder einem geringen Überschuss, vorzugsweise in einer Menge von 2,0 bis 2,5 mol auf ein mol Acetessigsäureester-magnesiumenolat, eingesetzt.
Die Acylierung des Acetessigsäureester-magnesiumenolats wird vorteilhaft in einem Lösungsmittel geringer bis mittlerer Polarität durchgeführt. Als Lösungsmittel werden vorzugsweise aromatische Kohlenwasserstoffe, wie Toluol oder Xylole, oder Ether, wie Dimethoxyethan, Tetrahydrofuran, Dioxan oder tert-Butyl-methylether, eingesetzt. Besonders bevorzugt ist Tetrahydrofuran.
Die Reaktion wird zweckmässig bei einer Temperatur von 20 bis 140°C durchgeführt, vorzugsweise bei 60 bis 90°C.
Die Abspaltung der Acetylgruppe aus dem als Zwischenprodukt entstehenden 2-Acyl-acetessigsäureester erfolgt vorteilhaft mit einem primären oder sekundären Amin oder vorzugsweise mit Ammoniak. Bei Verwendung von Ammoniak wird dieses vorzugsweise in einer Menge von 3 bis 5 mol, bezogen auf 1 mol des in der ersten Stufe eingesetzten Acetessigsäureester-magnesiumenolats, eingesetzt.
Die nachfolgenden Beispiele verdeutlichen die Durchführung des erfindungsgemässen Verfahrens.

### Beispiel 1

### Benzoylessigsäureethylester

Unter Stickstoff wurden 57,9 g (0,2 mol) Acetessigsäureethylester-magnesiumenolat in 750 ml Tetrahydrofuran aufgeschlämmt, 24,3 g (0,24 mol) Triethylamin zugegeben und 57,4 g (0,408 mol) Benzoylchlorid in 10 min zugetropft. Das Reaktionsgemisch wurde 2 h am Rückfluss gekocht, mit Eiswasser gekühlt und mit 285 ml 1N HCl hydrolysiert.
Die Phasen wurden getrennt und die Wasserphase mit tert-Butyl-methylether extrahiert. Die organischen Phasen wurden vereinigt und unter starkem Rühren wurde innerhalb von 10 min 140 ml 10%ige wässrige Ammoniaklösung zugegeben.
Nach 1 h wurden die Phasen getrennt, mit Na₂SO₄ getrocknet und eingeengt.
Der Rückstand wurde im Vakuum (0,1 mbar) destilliert.
Ausbeute: 63,7 g Benzoylessigsäureethylester mit einer Reinheit von 97,1%, entsprechend 78% der Theorie.
Siedepunkt: 85°C/0,1 mbar.

### Beispiel 2

### Pivaloylessigsäuremethylester (4,4-Dimethyl-3-oxopentansäuremethylester)

Unter Stickstoff wurden 51,7 g (0,2 mol) Acetessigsäuremethylester-magnesiumenolat in 730 ml Tetrahydrofuran aufgeschlämmt, 24,3 g (0,24 mol) Triethylamin zugegeben und 49,2 g (0,408 mol) Pivaloylchlorid in 10 min zugetropft.
Das Reaktionsgemisch wurde 2 h am Rückfluss gekocht, abgekühlt und mit 280 ml 1N HCl hydrolysiert.
Die Phasen wurden getrennt und die Wasserphase mit tert-Butyl-methylether extrahiert. Die organischen Phasen wurden vereinigt und unter starkem Rühren wurde innerhalb von 10 min 138 ml 10%ige Ammoniaklösung zugegeben. Nach 1 h wurden die Phasen getrennt, mit Na₂SO₄ getrocknet und eingeengt.
Der Rückstand wurde im Vakuum (13 mbar) destilliert.
Ausbeute: 52,4 g Methyl-pivaloylacetat mit einer Reinheit von 97,8%, entsprechend 81% der Theorie.
Siedepunkt: 66°C/13 mbar.

### Beispiel 3

### 4-Chlorbenzoylessigsäureethylester

Unter Stickstoff wurden 28,8 g (0,1 mol) Acetessigsäureethylester-magnesiumenolat in 375 ml Tetrahydrofuran aufgeschlämmt, 12,2 g (0,12 mol) Triethylamin zugegeben und 35,7 g (0,204 mol) 4-Chlorbenzoylchlorid in 15 min zugetropft. Das Reaktionsgemisch wurde 2 h am Rückfluss gekocht, mit Eiswasser gekühlt und mit 145 ml 1N HCl hydrolysiert.
Die Phasen wurden getrennt und die Wasserphase mit tert-Butylmethylether extrahiert. Die organischen Phasen wurden vereinigt und unter starkem Rühren wurde innerhalb von 15 min 70 ml 10%ige Ammoniaklösung zugegeben.
Nach 1 h wurden die Phasen getrennt, mit Natriumsulfat getrocknet und eingeengt.
Der Rückstand verfestigte sich beim Stehen. Das Produkt wurde aus Essigester/Hexan umkristallisiert.
Ausbeute: 39,0 g 4-Chlorbenzoylessigsäureethylester, entsprechend 86% der Theorie.
Schmelzpunkt: 37-38°C.

### Beispiel 4

### 2,4,5-Trifluorbenzoylessigsäureethylester

Unter Stickstoff wurden 7,2 g (0,025 mol) Acetessigsäureethylester-magnesiumenolat in 100 ml Tetrahydrofuran aufgeschlämmt, 3,1 g (0,03 mol) Triethylamin zugegeben und 10,0 g (0,05 mol) 2,4,5-Trifluorbenzoylchlorid in 10 min zugetropft. Das Reaktionsgemisch wurde 2 h am Rückfluss gekocht, mit Eiswasser gekühlt und mit 40 ml 1N HCl hydrolysiert.
Die Phasen wurden getrennt und die Wasserphase mit tert-Butylmethylether extrahiert. Die organischen Phasen wurden vereinigt und unter starkem Rühren wurde innerhalb von 10 min 20 ml 10%ige Ammoniaklösung zugegeben.
Nach 1 h wurden die Phasen getrennt, mit Natriumsulfat getrocknet und eingeengt.
Der Rückstand wurde im Vakuum (0,08 mbar) destilliert.
Ausbeute: 10,6 g 2,4,5-Trifluorbenzoylessigsäureethylester mit einer Reinheit von 98,0%, entsprechend 84% der Theorie.
Siedepunkt: 90°C/0,08 mbar.

### Beispiel 5

### 3-Oxo-5-phenyl-4-pentensäureethylester

Unter Stickstoff wurden 28,8 g (0,1 mol) Acetessigsäureethylester-magnesiumenolat in 375 ml Tetrahydrofuran aufgeschlämmt, 12,2 g (0,12 mol) Triethylamin zugegeben und 34,7 g (0,208 mol) Zimtsäurechlorid in 15 min zugetropft. Das Reaktionsgemisch wurde 2 h am Rückfluss gekocht, mit Eiswasser gekühlt und mit 145 ml 1N HCl hydrolysiert.
Die Phasen wurden getrennt und die Wasserphase mit tert-Butylmethylether extrahiert. Die organischen Phasen wurden vereinigt und unter starkem Rühren wurde innerhalb von 15 min 70 ml 10%ige Ammoniaklösung zugegeben.
Nach 1 h wurden die Phasen getrennt, mit Natriumsulfat getrocknet und eingeengt.
Der Rückstand wurde am Wasserstrahlvakuum (13 mbar) destilliert.
Ausbeute: 23,5 g 3-Oxo-5-phenyl-4-pentensäureethylester, entsprechend 54% der Theorie.
Siedepunkt: 140-142°C/13 mbar.

### Beispiel 6

### 3-Oxo-valeriansäuremethylester

Unter Stickstoff wurden 51,7 g (0,2 mol) Acetessigsäuremethylester-magnesiumenolat in 750 ml Tetrahydrofuran aufgeschlämmt, 24,3 g (0,24 mol) Triethylamin zugegeben und 38,9 g (0,408 mol) Propionylchlorid in 15 min zugetropft. Das Reaktionsgemisch wurde 2 h am Rückfluss gekocht, mit Eiswasser gekühlt und mit 285 ml 1N HCl hydrolysiert.
Die Phasen wurden getrennt und die Wasserphase mit tert-Butylmethylether extrahiert. Die organischen Phasen wurden vereinigt und unter starkem Rühren wurde innerhalb von 15 min 140 ml 10%ige Ammoniaklösung zugegeben.
Nach 1 h wurden die Phasen getrennt, mit Natriumsulfat getrocknet und eingeengt.
Der Rückstand wurde mit einer Spaltrohrkolonne bei 100 mbar destilliert.
Ausbeute: 23,9 g 3-Oxo-valeriansäuremethylester mit einer Reinheit von 96,0%, entsprechend 44% der Theorie.
Der als Vorlauf abgetrennte Acetessigsäuremethylester konnte wiederverwendet werden. Die Ausbeute erhöhte sich somit bezüglich umgesetztem Acetessigsäuremethylester auf 69%.
Siedepunkt: 109-111°C/100 mbar.

### Beispiel 7

### 3-Oxo-4-pentensäureethylester

Unter Stickstoff wurden 28,8 g (0,1 mol) Acetessigsäureethylester-magnesiumenolat in 350 ml Tetrahydrofuran aufgeschlämmt, 12,2 g (0,12 mol) Triethylamin zugegeben und 18,8 g (0,204 mol) Acryloylchlorid in 15 min zugetropft. Das Reaktionsgemisch wurde 2 h am Rückfluss gekocht, mit Eiswasser gekühlt und mit 75 ml 1N HCl hydrolysiert.
Die Phasen wurden getrennt und die Wasserphase mit tert-Butylmethylether extrahiert. Die organischen Phasen wurden vereinigt und unter starkem Rühren wurde innerhalb von 15 min 70 ml 10%ige Ammoniaklösung zugegeben.
Nach 1 h wurden die Phasen getrennt, mit Natriumsulfat getrocknet und eingeengt.
Der Rückstand wurde am Wasserstrahlvakuum (18 mbar) destilliert.
Ausbeute: 17,0 g 3-Oxo-4-pentensäureethylester mit einer Reinheit von 95,8%, entsprechend 57% der Theorie.
Siedepunkt: 78-80°C/18 mbar.

### Beispiel 8

### Benzoylessigsäure-tert-butylester

Unter Stickstoff wurden 34,4 g (0,1 mol) Acetessigsäure-tert-butylester-magnesiumenolat in 200 ml Tetrahydrofuran aufgeschlämmt, 12,2 g (0,12 mol) Triethylamin zugegeben und 29,0 g (0,24 mol) Benzoylchlorid in 15 min zugetropft. Das Reaktionsgemisch wurde 2 h am Rückfluss gekocht, mit Eiswasser gekühlt und mit 150 ml 1N HCl hydrolysiert.
Die Phasen wurden getrennt und die Wasserphase mit tert-Butylmethylether extrahiert. Die organischen Phasen wurden vereinigt und unter starkem Rühren wurden innerhalb von 15 min 70 ml 10%ige Ammoniaklösung zugegeben.
Nach 1 h wurden die Phasen getrennt, mit Natriumsulfat getrocknet und eingeengt.
Der Rückstand wurde im Vakuum (0,5 mbar) destilliert.
Ausbeute: 33,4 g Benzoylessigsäure-tert-butylester mit einer Reinheit von 97,4%, entsprechend 74% der Theorie.
Siedepunkt: 106-108°C/0,5 mbar.

### Beispiel 9

### Pivaloylessigsäurebenzylester

Unter Stickstoff wurden 41,5 g (0,1 mol) Acetessigsäurebenzylester-magnesiumenolat in 375 ml Tetrahydrofuran aufgeschämmt, 12,2 g (0,12 mol) Triethylamin zugegeben und 24,9 g (0,204 mol) Pivaloylchlorid in 15 min zugetropft. Das Reaktionsgemisch wurde 2 h am Rückfluss gekocht, mit Eiswasser gekühlt und mit 150 ml 1N HCl hydrolysiert.
Die Phasen wurden getrennt und die Wasserphase mit tert-Butylmethylether extrahiert. Die organischen Phasen wurden vereinigt und unter starkem Rühren wurde innerhalb von 15 min 70 ml 10%ige Ammoniaklösung zugegeben.
Nach 1 h wurden die Phasen getrennt, mit Natriumsulfat getrocknet und eingeengt.
Der Rückstand wurde im Vakuum (10 mbar) destilliert.
Ausbeute: 37,2 g Pivaloylessigsäurebenzylester mit einer Reinheit von 98,2%, entsprechend 78% der Theorie.
Siedepunkt: 156-158°C/10 mbar.

### Beispiel 10

### Benzoylessigsäureethylester

Zu einer Suspension von 57,9 g (0,2 mol) Acetessigsäureethylester-magnesiumenolat in 750 ml Tetrahydrofuran wurden unter Stickstoff 19,2 g (0,24 mol) Pyridin zugegeben und 57,4 g (0,408 mol) Benzoylchlorid in 10 min zugetropft. Das Reaktionsgemisch wurde 2 h am Rückfluss gekocht, mit Eiswasser gekühlt und mit 285 ml 1N HCl hydrolysiert.
Die Phasen wurden getrennt und die Wasserphase mit tert.-Butylmethylether extrahiert. Die organischen Phasen wurden vereinigt und unter starkem Rühren wurde innerhalb von 15 min 140 ml 10%ige wässrige Ammoniaklösung zugegeben.
Das Reaktionsgemisch wurde 1 h bei Raumtemperatur gerührt, die Phasen getrennt, mit Na₂SO₄ getrocknet und eingeengt.
Der Rückstand wurde im Vakuum (0,1 mbar) destilliert.
Ausbeute: 62,1 g Benzoylessigsäureethylester mit einer Reinheit von 97,5%, entsprechend 79% der Theorie.
Siedepunkt: 85°C/0,1 mbar.

### Beispiel 11

### Herstellung von Benzoylessigsäureethylester

Zu einer Suspension von 57,9 g (0,2 mol) Acetessigsäureethylester-magnesiumenolat in 750 ml Tetrahydrofuran wurden unter Stickstoff 29,4 g (0,24 mol) N,N-Dimethylanilin zugegeben und 57,4 g (0,408 mol) Benzoylchlorid in 10 min zugetropft. Das Reaktionsgemisch wurde 2 h am Rückfluss gekocht, mit Eiswasser gekühlt und mit 285 ml 1N HCl hydrolysiert.
Die Phasen wurden getrennt und die Wasserphase mit tert.-Butyl-methylether extrahiert. Die organischen Phasen wurden vereinigt und unter starkem Rühren wurde innerhalb von 15 min 140 ml 10%-ige wässrige Ammoniaklösung zugegeben.
Das Reaktionsgemisch wurde 1 h bei Raumtemperatur gerührt, die Phasen getrennt, mit Na₂SO₄ getrocknet und eingeengt.
Der Rückstand wurde im Vakuum (0,1 mbar) destilliert.
Ausbeute: 59,7 g Bezoylessigsäureethylester mit einer Reinheit von 97,8%, entsprechend der Theorie.
Siedepunkt: 85°C/0,1 mbar

### Beispiel 12

### Benzoylessigsäureethylester (Vergleichsbeispiel ohne Zugabe eines tertiären Amins)

Unter Stickstoff wurden 57,9 g (0,2 mol) Acetessigsäureethylester-magnesiumenolat in 700 ml Toluol aufgeschlämmt und 57,4 g (0,408 mol) Benzoylchlorid in 10 min zugetropft. Das Reaktionsgemisch wurde 3 h bei 80°C gerührt, mit Eiswasser gekühlt und durch Zugabe von 285 ml 1N HCl hydrolysiert.
Die Phasen wurden getrennt und die Wasserphase mit Toluol extrahiert. Die organischen Phasen wurden vereinigt, mit MgSO₄ getrocknet und eingeengt.
Der Rückstand wurde im Vakuum (0,1 mbar) destilliert.
Ausbeute: 33,2 g Benzoylessigsäureethylester mit einer Reinheit von 74,0%, entsprechend 32% der Theorie.
Das Nebenprodukt 2-Acetyl-acetessigsäureethylester liess sich destillativ nicht abtrennen.

### Beispiel 13

### Pivaloylessigsäuremethylester (Vergleichsbeispiel ohne Zugabe eines tertiären Amins)

Unter Stickstoff wurden 51,7 g (0,2 mol) Acetessigsäuremethylester-magnesiumenolat in 700 ml Toluol aufgeschlämmt und 49,2 g (0,408 mol) Pivaloylchlorid in 10 min zugetropft. Das Reaktionsgemisch wurde 3 h bei 80°C gerührt, mit Eiswasser gekühlt und durch Zugabe von 280 ml 1N HCl hydrolysiert.
Die Phasen wurden getrennt und die Wasserphase mit Toluol extrahiert. Die organische Phasen wurden vereinigt, mit MgSO₄ getrocknet und eingeengt.
Der Rückstand wurde im Vakuum (13 mbar) destilliert.
Ausbeute: 29,2 g Pivaloylessigsäuremethylester mit einer Reinheit von 78,4%, entsprechend 36% der Theorie.
Das Nebenprodukt 2-Acetyl-acetessigsäuremethylester liess sich destillativ nicht weiter abtrennen.

## Patentansprüche

1. Verfahren zur Herstellung von 3-Oxocarbonsäureestern der allgemeinen Formel 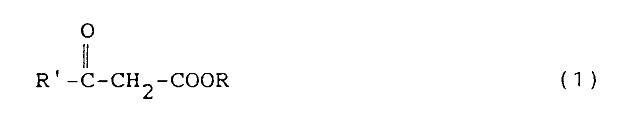 worin bedeuten
R ein geradkettiger oder verzweigter Alkylrest mit 1-20 C-Atomen,
ein Alkenyl- oder Alkinylrest mit 3-6 C-Atomen,
ein Cycloalkylrest,
ein mit einer Alkoxygruppe mit 1-4 C-Atomen substituierter Ethylrest,
ein mit einer gegebenenfalls substituierten Phenylgruppe substituierter Alkyl- oder Alkenylrest mit 1-4 C-Atomen,
ein gegebenenfalls substituierter Arylrest,
R' ein geradkettiger oder verzweigter Alkylrest mit 1-20 C-Atomen, der durch Halogene, niedrige Alkoxygruppen, niedrige Alkylthiogruppen, gegebenenfalls substituiertes Aryl, Cycloalkyl, Aryloxy, Arylthio oder gesättigte oder ungesättigte Heterocyclen substituiert sein kann, ausgenommen unsubstituiertes Methyl,
ein geradkettiger oder verzweigter Alkenylrest mit 2-20 C-Atomen, der durch Halogene oder Aryl substituiert sein kann,
ein Akinylrest, der durch Aryl substituiert sein kann,
ein gegebenenfalls substituierter Cycloalkyl- oder Cycloalkenylrest,
ein gegebenenfalls substituierter mono- oder polycyclischer Arylrest,
ein gegebenenfalls substituierter gesättigter oder ungesättigter mono- oder polycyclischer heterocyclischer Rest,
durch Acylierung der Magnesiumenolate der entsprechenden Acetessigsäureester der allgemeinen Formel 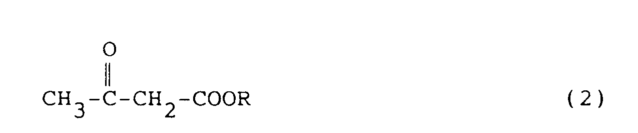 mit den entsprechenden Carbonsäurechloriden der allgemeinen Formel
R'-COCl (3)
zu den entsprechenden 2-Acyl-acetessigsäureestern der allgemeinen Formel 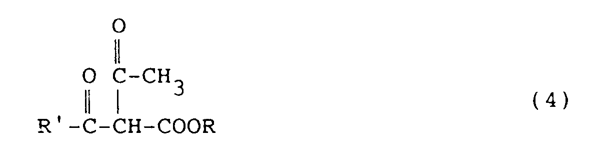 worin jeweils R und R' die oben genannten Bedeutungen haben, und nachfolgende Abspaltung der Acetylgruppe, dadurch gekennzeichnet, dass die Acylierung in Gegenwart eines tertiären Amins durchgeführt wird.

2. Verfahren nach Anspruch 1, dadurch gekennzeichnet, dass als tertiäres Amin eine Verbindung aus der Gruppe bestehend aus Trialkylaminen mit gleichen oder verschiedenen geradkettigen, verzweigten oder cyclischen Alkylresten, N-Alkylpyrrolidinen, N-Alkylpiperidinen, N-Alkylmorpholinen, Alkyl(arylalkyl)aminen, Aryl-alkyl-aminen, Chinuclidin, 1,4-Diazabicyclo[2.2.2]octan, Pyridin, Chinolin, Alkylpyridinen, 4-Dialkylaminopyridinen, 1,5-Diazabicyclo[4.3.0]non-5-en und 1,8-Diazabicyclo[5.4.0]undec-7-en oder ein Gemisch zweier oder mehrerer dieser Verbindungen eingesetzt wird.

3. Verfahren nach Anspruch 2, dadurch gekennzeichnet, dass als tertiäres Amin ein Trialkylamin eingesetzt wird.

4. Verfahren nach Anspruch 3, dadurch gekennzeichnet, dass als Trialkylamin Triethylamin eingesetzt wird.

5. Verfahren nach Anspruch 4, dadurch gekennzeichnet, dass, bezogen auf 1 mol des Acetessigsäureester-magnesiumenolats, das Carbonsäurechlorid in einer Menge von 2,0 bis 2,5 mol und das Triethylamin in einer Menge von 1,0 bis 1,4 mol eingesetzt wird.

6. Verfahren nach einem oder mehreren der Ansprüche 1 bis 5, dadurch gekennzeichnet, dass die Acylierung des Acetessigsäureester-magnesiumenolats in einem aliphatischen oder aromatischen Kohlenwasserstoff oder einem offenkettigen oder cyclischen Ether oder einem Gemisch solcher Verbindungen als Lösungsmittel durchgeführt wird.

7. Verfahren nach Anspruch 6, dadurch gekennzeichnet, dass als Lösungsmittel Tetrahydrofuran eingesetzt wird.

8. Verfahren nach einem oder mehreren der Ansprüche 1 bis 7, dadurch gekennzeichnet, dass die Acylierung des Acetessigsäureester-magnesiumenolats bei einer Temperatur von 20 bis 140°C durchgeführt wird.

9. Verfahren nach Anspruch 8, dadurch gekennzeichnet, dass die Acylierung bei einer Temperatur von 60 bis 90°C durchgeführt wird.

10. Verfahren nach einem oder mehreren der Ansprüche 1 bis 9, dadurch gekennzeichnet, dass die Abspaltung der Acetylgruppe mit einer Base aus der Gruppe Ammoniak, primäre Amine und sekundäre Amine erfolgt.

11. Verfahren nach Anspruch 10, dadurch gekennzeichnet, dass die Abspaltung der Acetylgruppe mit wässrigem Ammoniak erfolgt.

## Claims

1. A process for the manufacture of 3-oxocarboxylic acid esters having the general formula 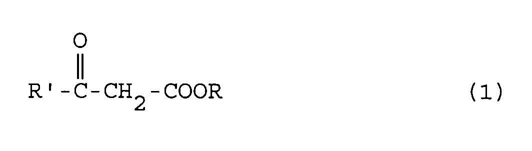 wherein
R is a straight-chain or branched alkyl radical having 1 to 20 C-atoms,
an alkenyl or alkynyl radical having 3 to 6 C-atoms,
a cycloalkyl radical,
an ethyl radical substituted with an alkoxy group having 1 to 4 C-atoms,
an alkyl or alkenyl radical having 1 to 4 C-atoms and being substituted with an optionally substituted phenyl group,
R' is a straight-chain or branched alkyl radical having 1 to 20 C-atoms, which may be substituted with halogens, lower alkoxy groups, lower alkylthio groups, optionally substituted aryl, cycloalkyl, aryloxy, arylthio, or saturated or unsaturated heterocycles, with the exception of unsubstituted methyl,
a straight-chain or branched alkenyl radical having 2 to 20 C-atoms which may be substituted with halogens or aryl,
an alkynyl radical which may be substituted with aryl,
an optionally substituted cycloalkyl or cycloalkenyl radical,
an optionally substituted mono- or polycyclic aryl radical,
an optionally substituted saturated or unsaturated mono- or polycyclic heterocyclic radical,
by acylation of the magnesium enolates of the corresponding acetoacetic acid esters having the general formula 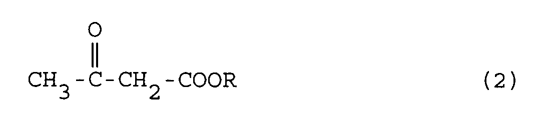 with the corresponding carboxylic acid chlorides having the general formula
R'-COCl (3)
to give the corresponding 2-acyl-acetoacetic acid esters having the general formula 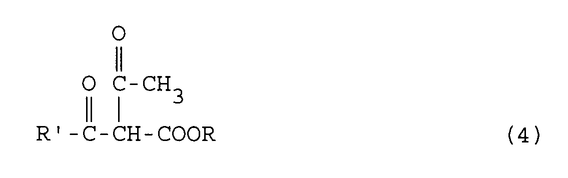 wherein each of R and R' have the above meanings, and subsequent cleavage of the acetyl group, characterized in that the acylation is carried out in the presence of a tertiary amine.

2. The process of claim 1, characterized in that as the tertiary amine a compound is used which is selected from the group consisting of trialkylamines having straight-chain, branched or cyclic alkyl radicals which may be the same or different from each other, N-alkylpyrrolidines, N-alkylpiperidines, N-alkylmorpholines, alkyl(arylalkyl)amines, aryl-alkyl-amines, quinuclidine, 1,4-diazabicyclo[2.2.2]octane, pyridine, quinoline, alkylpyridines, 4-dialkylaminopyridines, 1,5-diazabicyclo[4.3.0]non-5-ene and 1,8-diazabicyclo[5.4.0]undec-7-ene or a mixture of two or more of these compounds.

3. The process of claim 2, characterized in that as the tertiary amine a trialkylamine is used.

4. The process of claim 3, characterized in that as the trialkylamine triethylamine is used.

5. The process of claim 4, characterized in that the carboxylic acid chloride is used in an amount of from 2.0 to 2.5 moles and the triethylamine is used in an amount of from 1.0 to 1.4 moles, based on 1 mole of the magnesium enolate of the acetoacetic acid ester.

6. The process of one or more of the claims 1 to 5, characterized in that the acylation of the magnesium enolate of the acetoacetic acid ester is carried out in an aliphatic or aromatic hydrocarbon or in an open-chain or cyclic ether or in a mixture of these compounds as a solvent.

7. The process of claim 6, characterized in that tetrahydrofuran is used as the solvent.

8. The process of one or more of the claims 1 to 7, characterized in that the acylation of the magnesium enolate of the acetoacetic acid ester is carried out at a temperature of from 20 to 140°C.

9. The process of claim 8, characterized in that the acylation is carried out at a temperature of from 60 to 90°C.

10. The process of one or more of the claims 1 to 9, characterized in that the cleavage of the acetyl group is effected with a base selected from the group consisting of ammonia, primary amines and secondary amines.

11. The process of claim 10, characterized in that the cleavage of the acetyl group is effected with aqueous ammonia.

## Revendications

1. Procédé Pour la préparation d'esters de l'acide 3-oxocarboxylique de la formule générale 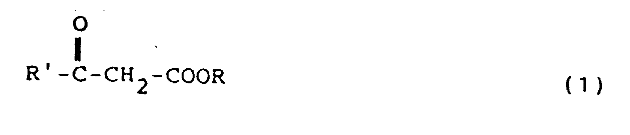 dans laquelle
R signifie un radical alcoyle linéaire ou ramifié avec 1-20 atomes C,
un radical alcényle ou alkinyle avec 3-6 atomes C,
un radical cycloalkyle,
un radical éthyle substitué avec un groupe alcoxy comportant 1-4 atomes C,
un radical alkyle ou alcényle substitué par un groupe phényle éventuellement substitué, radical alkyle ou alcényle ayant 1-4 atomes C,
un radical aryle éventuellement substitué,
R' signifie un radical alkyle linéaire ou ramifié avec 1-20 atomes C, qui peut être substitué par halogène, des groupes alcoxy inférieurs, des groupes alkylthio inférieurs, éventuellement aryle substitué, cycloalkyle, aryloxy, arylthio ou des hétérocycles saturés ou insaturés, à l'exception du méthyle non substitué,
un radical alcényle ou linéaire ou ramifié avec 2-20 atomes C qui peut être substitué par halogène ou aryle, un radical alkynyle qui peut être substitué par aryle, un radical cycloalkyle ou cycloalcényle, éventuellement substitué,
un radical aryle mono- ou polycyclique éventuellement substitué,
un radical mono- ou polycyclique, hétérocyclique saturé ou insaturé éventuellement substitué,
par acylation des magnésiuménolates des esters de l'acide acétylacétique correspondant de la formule générale 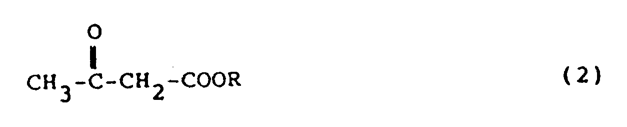 avec les chlorures de l'acide carboxylique correspondants de la formule générale
R'-COCl (3)
aux esters de l'acide 2-acyl-acétylacétique correspondants de la formule générale 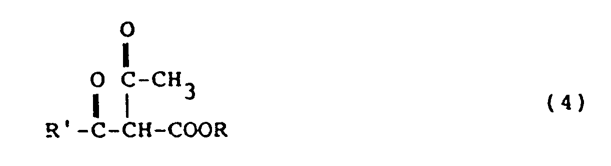 dans laquelle chaque fois R et R' ont les significations précitées et séparation consécutive du groupe acétyle, caractérisé en ce que l'acylation est conduite en présence d'une amine tertiaire.

2. Procédé selon la revendication 1, caractérisé en ce qu'en tant qu'amine tertiaire, on fait appel à un composé à partir du groupe constitué par des trialkylamines avec les radicaux identiques ou différents linéaires, ramifiés ou cycliques N-alkylpyrrolidines, N-alkylpipéridines, N-alkylmorpholines, alkyl(arylalkyl)amines, aryl-alkyl-amines, quinuclidines, 1,4-diazabicyclo[2,2,2]octane, pyridine, quinoline, alkylpyridines, 4-dialkylaminopyridines, 1,5-diazabicyclo[4,3,0]non-5-ène et 1,8-diazabicyclo[5,4,0]undec-7-ène ou un mélange de deux, voire davantage de ces composés.

3. Procédé selon la revendication 2, caractérisé en ce qu'en tant qu'amine tertiaire, on met en oeuvre une trialkylamine.

4. Procédé selon la revendication 3, caractérisé en ce qu'en tant que trialkylamine, on met en oeuvre une triéthylamine.

5. Procédé selon la revendication 4, caractérisé en ce qu'on met en oeuvre rapporté à un mole de l'ester de l'acide acétylacétique-magnésiuménolate, le chlorure de l'acide carboxylique dans une quantité de 2,0 à 2,5 moles et la triéthylamine dans une quantité de 1,0 à 1,4 moles.

6. Procédé selon l'une ou plusieurs des revendications 1 à 5, caractérisé en ce que l'acylation de l'ester de l'acide acétylacétique-magnésiuménolate est conduite dans un hydrocarbure aliphatique ou aromatique ou dans un éther cyclique ou à chaîne ouverte ou dans un mélange de ces composés en tant que solvant.

7. Procédé selon la revendication 6, caractérisé en ce que l'on met en oeuvre du tétrahydrofuranne en tant que solvant.

8. Procédé selon l'une ou plusieurs des revendications 1 à 7, caractérisé en ce que l'acylation de l'ester de l'acide acétylacétique-magnésiuménolate est conduite à une température de 20 à 140°C.

9. Procédé selon la revendication 8, caractérisé en ce que l'acylation s'effectue à une température de 60 à 90°C.

10. Procédé selon l'une ou plusieurs des revendications 1 à 9, caractérisé en ce que la séparation du groupe acétyle s'effectue avec une base à partir du groupe ammoniaque, amine primaire et amine secondaire.

11. Procédé selon la revendication 10, caractérisé en ce que la séparation du groupe acétyle s'effectue avec de l'ammoniaque aqueuse.
